# EUROPEAN PATENT APPLICATION

(11) **EP 0 694 283 A2**
(43) Date of publication of application: **31.01.1996**
(21) Application number: 95111548.4
(22) Date of filing: 22.07.1995
(51) Int. Cl.: A61B 5/00

(54) **System for measuring the pulse rate and/or the blood pressure during a telephone call**

(30) Priority: 24.07.1994 EP 94111543
(71) Applicant: AUSTEL Licensing GmbH, A-5020 Salzburg (AT)
(72) Inventor: De Brun, Cathal, Coolock Dublin 17 (IE); Savery, Win, Jamesburg, N.J. 08831 (US)
(74) Representative: Kaminski, Susanne, Dr.

(57) **Abstract**

A system for monitoring the pulse beat and/or the blood pressure while being able to have a telephone conversation, includes the following features:
- a telephone set;
- pulse detection means and/or blood pressure detection means that are incorporated into the telephone connected to at least one sensor, sensoring the pulse beat and/or the blood pressure; and
- first informing and/or alerting means, coupled to the pulse detection means and/or blood pressure detection means. Furthermore, there may be comprised finger detection means for detecting the contact of the finger with the at least one sensor and/or a movement transducer with noise cancelling system.

## Description

The present invention refers to a system for monitoring the pulse beat and/or the blood pressure of a person while on the telephone.

There are known a variety of pulse beat sensors or pulsimeters or a plurality of means for non-invasive monitoring the blood pressure. From EP-A 0 410 658 there is known, for example, a pulsimeter comprising a pulse detection circuit for detecting a pulsation and for outputting a corresponding pulse signal and a pulsation calculation means for calculating a pulse rate in response to the pulse signal. There are further provided pulse width evaluation means for evaluating the width of the pulse and for distinguishing a signal referring to a real pulse signal from a signal resulting from noise of the device.

EP-A 0 559 203 describes a pulsimeter being coupled with a microcomputer, wherein pulses detected by an earlobe sensor are stored in the microcomputer during a predetermined time interval allowing the calculation of consumed energy or consumed calories during the measuring time intervals. This known device is rather complicated and coupled with an exercise device for enabling a proper evaluation of the amount of exercise during a predetermined time interval.

From EP-A 0537 383 there is known an inflatable finger cuff for use in non-invasive monitoring the instantaneous blood pressure, wherein a finger of a patient is encircled by the inflatable finger cuff. For avoiding a drift in the mean level of the light detector signal emitted by the sensor over a long period of time the light source and the light detector are located within an inflatable space between a rigid cylinder and a compliant tube of the finger cuff, wherein the tube is made of translucent material in this known device. Such a device is very complicated to handle and necessitates a very large and expensive evaluating and monitoring unit.

There are some documents describing the transmission of signals relating to the blood pressure and/or the heart rate of persons by using telephone equipments. The device of EP-A1-237 588 includes means for generating a signal representing the blood pressure of a patient as well as a signal representing the identity of this patient. Those signals are generated in a format suitable for telephonic transmission. The signals are then transmitted to a remote central data collecting and processing system, wherein they are compared to the specific partient's nominal values. The central data collecting and processing system may activate signal means indicating to the patient or to the patient's physician that immediate attention is advisable.

The system according to EP-A1-447 710 includes an electrocardiograph detecting the heart beats and transmitting the respective electrocardiographic signals via telephone line to an electrocardiographic analyzer of a hospital, thus enabling the diagnosis of the circulatory functions of the specific person.

The apparatus according to EP-A2-122 888 comprises an instrument for monitoring a patient's electrocardiographic signals, which signals are fed via an acoustic transducer to the microphone of a telephone. At the receiving end of the telephone line the incoming signals are demodulated and/or graphically recorded, in order as to enable a cardiologist to make a diagnosis and to give a relevant advice.

Although such devices might be of advantage for risk patients who appreciate and need, in cases, a control facility around the clock and quick action by a specialist's care, also other persons, who themselves are not included in this group, may be interested in having a simple control facility for their heart frequency.

The psychological condition, called manager syndrome, of persons who are permanently short of time, are continuously under the pressure to be successful and are hardly unstressed can cause a tendency to tachicardia, to signs of cardial insufflciency and even to warning indications of a tendency to myocardial infarct.

It is an object of the present invention to provide an apparatus enabling persons, such as managers in particular, who pass a good deal of their time at telephone, to effect a kind of self-control of their heartbeat or to keep their temper under control during difficult and psychologically stressing business talks or even private talks, particularly as a communication promoting aid.

This object is attained by realizing the characterizing features of claim 1. Futher alternative and/or advantageous constructions in accordance with the invention are defined by the characterizing features of the subclaims.

It is therefore an object of the present invention to provide a device for monitoring the pulse beat and/or the blood pressure of a person which is easy to use while being on the telephone and does not require a troublesome process for applying a monitoring or detecting device.

The present invention provides a simple system for monitoring the pulse beat and/or the blood pressure of a person while being on the telephone. When making a telephone call these values mentioned above can rapidly change with changing emotions of the caller. Thereby effective monitoring can be provided too and - in cases of emergency - alarming means may be activated.

By incorporating the pulse detection means and/or blood pressure detection means into a telephone set the inventive system is easy to use and to handle and it is also possible to check the pulse beat and/or the blood pressure of a person any time the telephone is used. To make the unit work one would only have to put a finger, preferably the thumb, in a corresponding recess, respectively on a correspondingly adapted region provided on the telephone and the pulse beat and/or the blood pressure are measured. When, because of changing emotions during the telephone conversation, the measured value reaches some predetermined threshold level this condition can be indicated and there can also be given a warning signal.

For handling emergency cases the first informing and/or alerting means may be coupled with an automatic dialling mechanism for dialling preprogrammed telephone numbers according to an other embodiment of the present invention. Thereby the telephone conversation being held at this same time and possibly causing the emergency values of the pulse beat and/or the blood pressure is interrupted and an emergency number will automatically be dialled allowing a remote first aid organisation of effective rescue operations.

In particular for elderly or ill persons it may be useful that their pulse beat and/or blood pressure are regularly checked by experts and this feature can be very easily achieved with the inventive system without hindering them while working. For this purpose there may be provided at least one further telephone line, which is to be activated automatically in case of such emergencies without the need of interrupting the person's telephone conversation and without causing any uneasiness by alarming him. Therefore, the pulse detection means and/or blood pressure detection means are directly coupled via this second telephone line to a health or care center. In this way, each time the person to be controlled is making a telephone call, in case the measured data should be alarming these data are transmitted to an electrocardiographic center wherein these data are compared to the person's reference data (the correct relation is done via the registered telephone number). In case of really alarming values an alarm signal may be transmitted to the person via the telephon asking the person to get in contact with his doctor in charge.

In order to ensure that a correct signal of the pulse beat and/or the blood pressure is taken the inventive system may further comprise at least one of the following features:
- finger detection means or detecting the contact of the finger with the pulse detection means and/or the blood pressure detection means; and
- a movement transducer with noise cancelling system.

With the finger detection means it is possible to easily check whether the finger is correctly inserted into the respective recess being provided with a corresponding sensor. Furthermore, the movement transducer with noise cancelling system allows a simple correction of errors which could be induced by a movement of the finger during the measuring process.

For giving a clear indication whether the finger is inserted correctly into the recess comprising the measuring sensor the finger detection means is coupled with second informing and/or alerting means according to a further preferred embodiment of the present invention. These second informing and/or alerting means give a signal, if the finger is not correctly inserted and may induce the user of the telephone to position his finger in a correct position.

For being able to use the inventive system in existing telephone sets the pulse detection means and/or the blood pressure detection means may be provided in the main body of said telephone, and the pressure sensor means may advantageously be located in the handset of the telephone. Such pressure sensor means can be easily integrated into the handset of the telephone without greatly modifying the design and there must only be provided a respective recess for inserting a finger. The detection and signal evaluating means can be integrated into the main body of the telephone and existing displays of the telephone may also be used as informing and/or alerting means of the inventive system. Thereby the additional components for installing the inventive system for monitoring the pulse beat and/or the blood pressure are reduced to a minimum.

The inventive system for controlling the pulse beat and/or the blood pressure of a person may be used for monitoring the momentary values of the person using the telephone. The inventive system may, however, also be used monitoring and comparing these values over a longer period of time and/or for several persons. Therefore, according to a specific embodiment of the present invention, the system further comprises a memory for entering measured data, the memory being integrated into the main body of the telephone. An integrated clock may furnish correlative time- and date-values.

If one telephone is to be used by different persons the inventive system may further comprise input means for feeding in set-points and predetermined band widths - of admissible pulse frequencies - respectively. Each user may enter a code for identifying himself to the system. Thereafter the system is using the respective preprogrammed set-points and band widths and will compare the momentary data with stored values from earlier measurements. Such identification code may also guarantee that only an authorized person can use the telephone and/or have access to the data stored in the memory of the inventive system for monitoring the pulse beat and/or the blood pressure.

With a simple construction the input means are preferably the keys of the telephone. For an easy association of the data the memory comprises an idexing register and/or indexing means for storing the data in relation to predetermined values, such as person, time, date, resting value etc. according to a further embodiment of the present invention. In order to facilitate the access, memory locations addressable by personal code may be installed for case of finding them again.

As several types of telephones are combined with printers or telefax devices these features can also be used in connection with the inventive system for monitoring the pulse beat and/or the bloodpressure wherein printing means are provided for printing out data of the momentary pulse value and/or the blood pressure and for printing out the stored and indexed data. Such a print, which optionally is only enabled after inputting a code, may then include indications of time and date assigned to each of the values registered and should only be possible in a person related manner.

These and other characteristic features and advantages of the present invention will also become apparant from the following description of an exemplary and preferred embodiment of the inventive system for monitoring the pulse beat and/or the blood pressure being shown in the enclosed drawing, wherein:
- Fig.1: is a schematic view of a telephone incorporating the inventive system for monitoring the pulse beat and/or the blood pressure;
- Fig.2: is a schematic diagram of the essential commponents of the system for monitoring the pulse beat and/or the blood pressure of a person according to the present invention; and
- Fig.3: shows an inventive system in use.

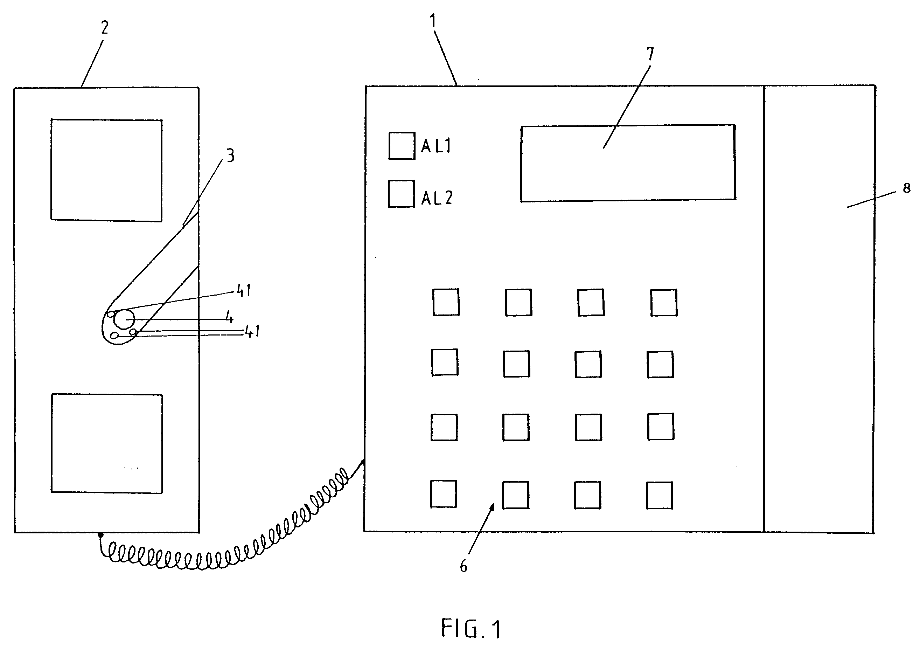

In Fig. 1 reference numeral 1 designates the main body of a telephone and with 2 the handset of the telephone. The handset 2 is provided with a recess 3 for receiving a finger, in particular the thumb, of a user of the telephone.

Within the recess 3 there is provided a sensor 4 for measuring the pulse beat and/or the blood pressure, when a finger is received in the recess 3. The sensor 4 also comprises finger detection means 41 for detection the contact of the finger, which may be formed by pressure sensors, beat sensors or as sensors using the skin-effect. Instead of the line 5 between the handset 2 and the main body 1 of the telephone there can also be provided a wireless connection between these components as is widely known per se.

The main body 1 comprises a plurality of keys generally indicated with 6 and a display portion 7. Furthermore there are provided first alarm or alerting means AL1 and second alarm or alerting means AL2, whose function will be described in more detail below. The main body 1 of the telephone is further coupled with a printer 8, which may be only a printing device or which may also incorporate a telefax device. Such devices are widely known and are, therfore, not further described.

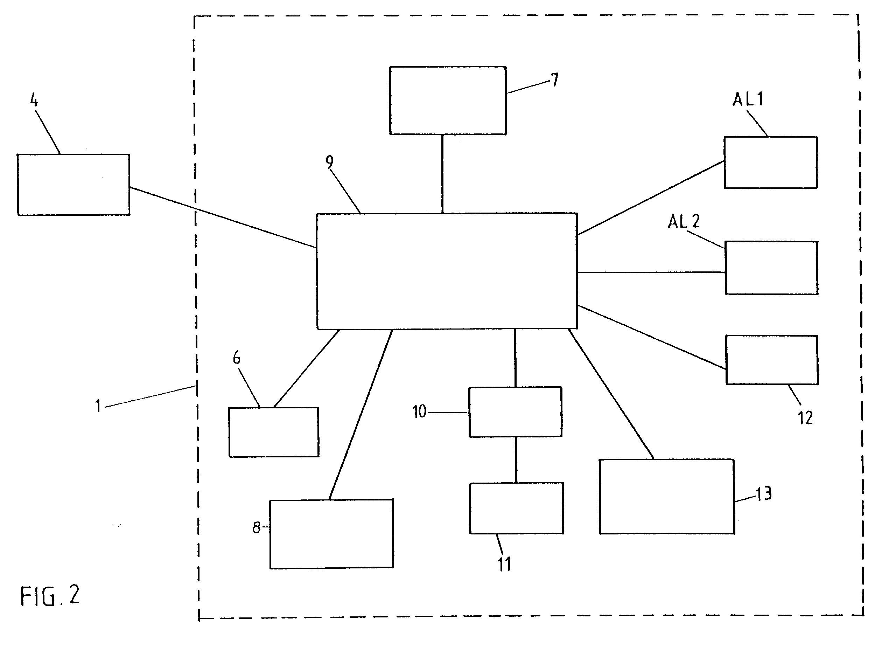

In the schematic diagram of Fig. 2 the reference numerals of Fig. 1 are used for identical components. The sensor 4 being incorporated in the handset of the telephone is coupled with the actual pulse detection and/or blood pressure detection means 9, in in which the signals of the sensor 4 are converted into values related to the pulse beat and/or the blood pressure of the person using the telephone. The measured values can be indicated in the display portion 7 of the telephone and can also be printed with the printer 8.

If the measured values for the pulse beat and/or the blood pressure given by the unit 9 reach a certain value, which may be preprogrammed according to individual aspects, an alarm signal is produced, which can be indicated by the alerting means AL1.

The finger detection means 41 (Fig.1) for detecting the contact of the finger induce an indication and an alarm signal, if there is no sufficient contact between the finger inserted into the recess of the handset 2 and the sensor 4 for detecting the pulse beat and/or the blood pressure of the user of the telephone. This alarm signal is indicated by the second alerting means AL2 and can also be shown on the display 7.

The unit 9 being the actual pulse detection means and/or blood pressure detection means are further coupled with a memory 10 being also integrated into the main body 1 of the telephone. This memory serves for storing measured data and also for storing set-points, predetermined band widths for the signals to be measured as well as threshold values for inducing alarm signals. Such predeterminded or preproprammed values are entered into the memory 10 through the keys 6 of the telephone. If the memory is subdivided, quick addressing may be effected by codes inputted via the keys, as has been indicated above. Furthermore, the memory 10 can be coupled. Furthermore, the memory 10 can be coupled with an indexing register and/or indexing means 11 for indexing and for storing the data in relation to predetermined values, such as person, time, date, resting value etc.. In this way there may be stored the measured values for different users of the telephone and there can also be provided a control over long periods of time.

In cases of emergency it might be useful to inform paricular persons or emergency units of an impending heart attack, for example, or a similar condition. Therefore there is also provided an automatic dialling mechanism 12 for dialling preprogrammed telphone numbers in case of an emergency, if the measured values reach certain threatening threshold values. In this case the actual telephone conversation will normally not be interrupted as a special line is provided only for this case and there will be automatically sent a telephone call to a particular person and/or an emergency center informing about the bad health of the user of the telephone.

Furthermore, the pulse detection means and/or blood pressure detection means 9 are coupled with a transmitting device 13 for transmitting the measured data to a health or care center. In this way, each time an elderly or handicapped person to be controlld by a health center or a relative is making a telephone call with a health or care center, the measured data are transmitted to the center and these values can be checked without the necessity that these persons are visited each time by a nurse or physician. This system is widely in accordance with a system as known from the EP-A1-237588 for instance.

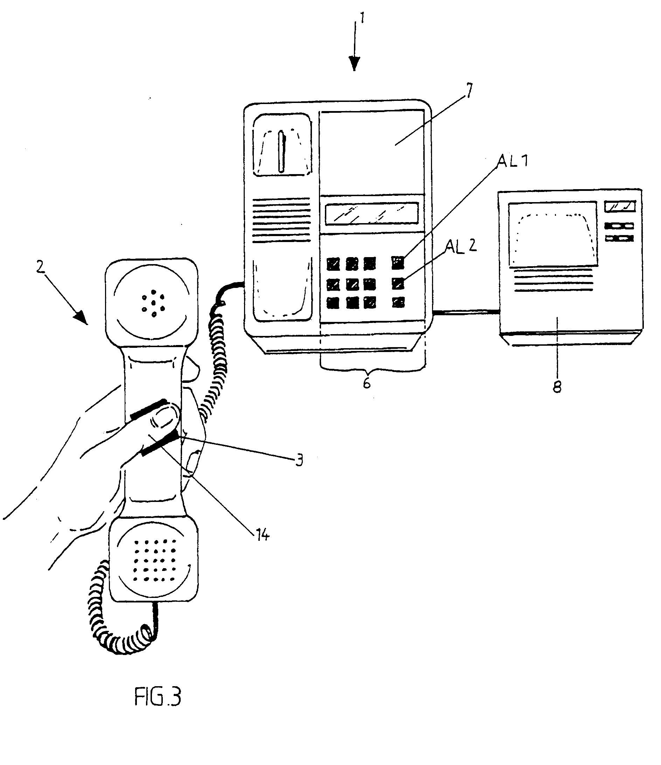

Fig.3 shows a device according to the invention in which the thumb 11 of a person is laid into the recess 3 of a handset 2 which is a natural positon in handling a telephone, as may be seen. The main body 1 of the telephone set corresponds substantially to the usual construction. Operation is effected via the individual keys 6 of the key board. The corresponding value may be shown on the display 7, but the user is free to activate the representation via the display 7 or not by one of the keys appropriately provided for this purpose.

The first and second alerting mesans AL1 and AL2 may be designed as blinking or flashing keys or lamps, but an accoustic signal is also conceivable.

To the main body 1, a printer 8 is connected by which the data obtained may optionally be printed out. The reference data can either be read in from a PC via an interface provided with the telephone, or may be directly inputted as a mean value of a plurality of measurements of the pulse in rest into the memory. This may be done by an appropriate program via the microprocessor, optionally supported by a menu on display 7.

## Claims

1. A system for monitoring the pulse beat and/or the blood pressure while being able to have a telephone conversation, including the following features:
- a telephone set;
- pulse detection means and/or blood pressure detection means that are incorporated into the telephone connected to at least one sensor, sensoring the pulse beat and/or the blood pressure; and
- first informing and/or alerting means, coupled to the pulse detection means and/or blood pressure detection means.

2. A system according to claim 1, further comprising at least one of the following features:
- finger detection means for detecting the contact of the finger with the at least one sensor; and
- a movement transducer with noise cancelling system.

3. A system according to claim 2, wherein the sensor is coupled with second informing and/or alerting means.

4. A system according to claims 1, 2 or 3, wherein the pulse detection means and/or the blood pressure detection means are provided in the main body of said telephone, and wherein the at least one sensor, eventually in the form of a pressure sensor - is located in the handset of the telephone.

5. A system according to any of the preceding claims, further comprising a memory for entering measured data, the memory being integrated into the main body of the telephone.

6. A system according to claim 5, further comprising input means for feeding in set-points and predetermined band widths, respectively.

7. A system according to claim 6, wherein the input means are the keys of the telephone.

8. A system according to claims 5, 6 or 7, wherein the memory comprises an indexing register and/or indexing means for storing the data in relation to predetermined values, such as person, time, date, resting value etc..

9. A system according to any of the preceding claims, wherein printing means are provided for printing out data of the momentary pulse value and/or the blood pressure and for printing out the stored and indexed data.

10. A system according to any of the preceding claims, wherein the pulse detection means and/or blood pressure detection means and/or the first informing and/or alerting means are coupled with an automatic dialling mechanism for dialling preprogrammed telephone numbers in case of an emergency, and whereby preferably at least one further telephone line is provided for transmitting the measured data to a health or care center.
